# EUROPEAN PATENT SPECIFICATION

(11) **EP 3 033 995 B1**
(45) Date of publication and mention of the grant of the patent: **05.06.2019**
(21) Application number: 15200942.9
(22) Date of filing: 17.12.2015
(51) Int. Cl.: A61B 5/042, A61B 5/053

(54) **FAR FIELD-INSENSITIVE INTRACARDIAC CATHETER ELECTRODES**
WEITFELD-UNEMPFINDLICHE INTRAKARDIALE KATHETERELEKTRODEN
ÉLECTRODES DE CATHÉTER INTRACARDIAQUE INSENSIBLES AUX CHAMPS LOINTAINS

(30) Priority: 18.12.2014 US 201462093773 P; 02.11.2015 US 201514929990
(43) Date of publication of application: 22.06.2016
(73) Proprietor: Biosense Webster (Israel) Ltd., 2066717 Yokneam (IL)
(72) Inventor: LEVIN, Michael, 3559017 Haifa (IL); BAR-TAL, Meir, 3224009 Haifa (IL); ULTCHIN, Yigal, 7628810 Rehovot (IL)
(74) Representative: Carpmaels & Ransford LLP

(56) References cited:
- WO-A1-2014/047068
- WO-A1-2014/085405
- WO-A2-00/57947

## Description

### CROSS-REFERENCE TO RELATED APPLICATIONS

This Application claims the benefit of U.S. Provisional Application No. 62/093,773, filed 18 December 2014.

### BACKGROUND OF THE INVENTION

### 1. Field of the Invention.

This invention relates to cardiac physiology. More particularly, this invention relates to the evaluation of electrical propagation in the heart.

### 2. Description of the Related Art.

Cardiac arrhythmias such as atrial fibrillation are an important cause of morbidity and death. Commonly assigned U.S. Patent No. 5,546,951, and U.S. Patent No. 6,690,963, both issued to Ben Haim and PCT application WO 96/05768, disclose methods for sensing an electrical property of heart tissue, for example, local activation time, as a function of the precise location within the heart. Data are acquired with one or more catheters having electrical and location sensors in their distal tips, which are advanced into the heart. Methods of creating a map of the electrical activity of the heart based on these data are disclosed in commonly assigned U.S. Patent No. 6,226,542, and U.S. Patent No. 6,301,496, both issued to Reisfeld. As indicated in these patents, location and electrical activity is typically initially measured on about 10 to about 20 points on the interior surface of the heart. These data points are then generally sufficient to generate a preliminary reconstruction or map of the cardiac surface. The preliminary map is often combined with data taken at additional points in order to generate a more comprehensive map of the heart's electrical activity. Indeed, in clinical settings, it is not uncommon to accumulate data at 100 or more sites to generate a detailed, comprehensive map of heart chamber electrical activity. The generated detailed map may then serve as the basis for deciding on a therapeutic course of action, for example, tissue ablation, to alter the propagation of the heart's electrical activity and to restore normal heart rhythm.

Catheters containing position sensors may be used to determine the trajectory of points on the cardiac surface. These trajectories may be used to infer motion characteristics such as the contractility of the tissue. As disclosed in U.S. Patent No. 5,738,096, issued to Ben Haim, maps depicting such motion characteristics may be constructed when the trajectory information is sampled at a sufficient number of points in the heart.

Electrical activity at a point in the heart is typically measured by advancing a multiple-electrode catheter to measure electrical activity at multiple points in the heart chamber simultaneously. A record derived from time varying electrical potentials as measured by one or more electrodes is known as an electrogram. Electrograms may be measured by unipolar or bipolar leads, and are used, e.g., to determine onset of electrical propagation at a point, known as local activation time.

Sensors in a cardiac chamber may detect far-field electrical activity, i.e., the ambient electrical activity originating away from the sensors, which can distort or obscure local electrical activity, i.e., signals originating at or near the sensor location. Commonly assigned U.S. Patent Application Publication No. 2014/0005664 of Govari et al.*,* discloses distinguishing a local component in an intracardiac electrode signal, due to the tissue with which the electrode is in contact from a remote-field contribution to the signal, and explains that a therapeutic procedure applied to the tissue can be controlled responsively to the distinguished local component.

U.S. Patent Application Publication No. 2014/0187991 of Thakur et al. proposes a method for mapping a cardiac chamber by sensing activation signals of intrinsic physiological activity with a plurality of electrodes disposed in or near the cardiac chamber. The method includes isolating R-wave events in the activation signals, generating a far-field activation template representative of a far-field activation signal component based on the R -wave events, and filtering the far-field activation template from the activation signals to identify near-field activation signal components in the activation signals.

Receiving electrogram signals from intracardiac catheters is complicated by undesirable far field signal component mixed with near field electrical signals. In this environment near field signals indicate local activation, i.e., propagation of a signal through a local regions being sensed by the electrodes. Detection of local activation is widely employed as an electrophysiological indicator of the local state of the heart. The far field electrical signals contain no useful information about local heart activation and only disturb the measurements.

WO 00/57947 describes a bipolar sensor for muscle tissue action potential duration estimation.

WO 2014/085405 A1 describes a catheter system for measuring electrical properties of tissue.

WO 2014/047068 A1 describes a map and ablate closed-loop cooled ablation catheter.

### SUMMARY OF THE INVENTION

The negative influence of the far field signals increases with the distance between the measuring intracardiac electrodes and the endocardium. Although the use of bipolar electrode configurations mitigates the effect, in many kinds of electrophysiological studies it is important to measure unipolar local activation potentials.

According to disclosed embodiments of the invention, guard electrodes are placed around the measuring electrodes of a cardiac catheter.

There is provided according to embodiments of the invention an apparatus including a catheter, an electrode assembly disposed on the distal portion of the catheter. The electrode assembly includes a sensing electrode connected to a remote receiver, and at least one grounded guard electrode spaced apart from the sensing electrode by a gap. The sensing electrode is bracketed on at least two sides by the at least one grounded guard electrode.

According to still another aspect of the apparatus, the sensing electrode has a width dimension, and the gap is one-half the width dimension.

According to an additional aspect of the apparatus, the sensing electrode and the at least one grounded guard electrode are ring electrodes that encircle the circumference of the distal portion of the catheter.

According to another aspect of the apparatus, the sensing electrode and the at least one grounded guard electrode are elevated above the external surface of the catheter.

According to one aspect of the apparatus, the sensing electrode and the at least one grounded guard electrode have respective width dimensions, wherein elevations of the sensing electrode and the at least one grounded guard electrode above the external surface of the catheter are less than the respective width dimensions.

According to one aspect of the apparatus the sensing electrode and the at least one grounded guard electrode are flush with the external surface of the catheter.

According to a further aspect of the apparatus, the at least one grounded guard electrode is a single electrode that surrounds the sensing electrode.

There is further provided a method, which is carried out by inserting into a heart of a living subject a catheter having an electrode assembly disposed on the distal portion of the catheter. The electrode assembly includes a sensing electrode connected to a remote receiver, and at least one grounded guard electrode spaced apart from the sensing electrode by a gap. The sensing electrode is bracketed on at least two sides by the at least one grounded guard electrode. The method is further carried out by receiving signals from the sensing electrode in the receiver.

### BRIEF DESCRIPTION OF THE SEVERAL VIEWS OF THE DRAWINGS

For a better understanding of the present invention, reference is made to the detailed description of the invention, by way of example, which is to be read in conjunction with the following drawings, wherein like elements are given like reference numerals, and wherein:
Fig. 1 is a pictorial illustration of a system for detecting electrical activity in a heart of a living subject in accordance with an embodiment of the invention;
Fig. 2 is a schematic diagram of an electrode assembly on the shaft of a cardiac catheter in accordance with an embodiment of the invention;
Fig. 3 is a schematic sectional view through the longitudinal axis of the distal portion of a cardiac catheter in accordance with an embodiment of the invention;
Fig. 4 is a schematic side view of an electrode arrangement on the distal portion of a cardiac catheter in accordance with an embodiment of the invention;
Fig. 5 is a schematic plan view of an electrode arrangement on the distal portion of a cardiac catheter in accordance with an alternate embodiment of the invention; and
Fig. 6 - Fig. 17 are simulation examples showing the effect on the far field component of an intracardiac electrogram as the height of the guard electrodes varies from 0.2 - 10 mm.

### DETAILED DESCRIPTION OF THE INVENTION

In the following description, numerous specific details are set forth in order to provide a thorough understanding of the various principles of the present invention. It will be apparent to one skilled in the art, however, that not all these details are necessarily needed for practicing the present invention. In this instance, well-known circuits, control logic, and the details of computer program instructions for conventional algorithms and processes have not been shown in detail in order not to obscure the general concepts unnecessarily. The invention is defined in the appended claims, the description is for only illustrative purposes.

### System Overview.

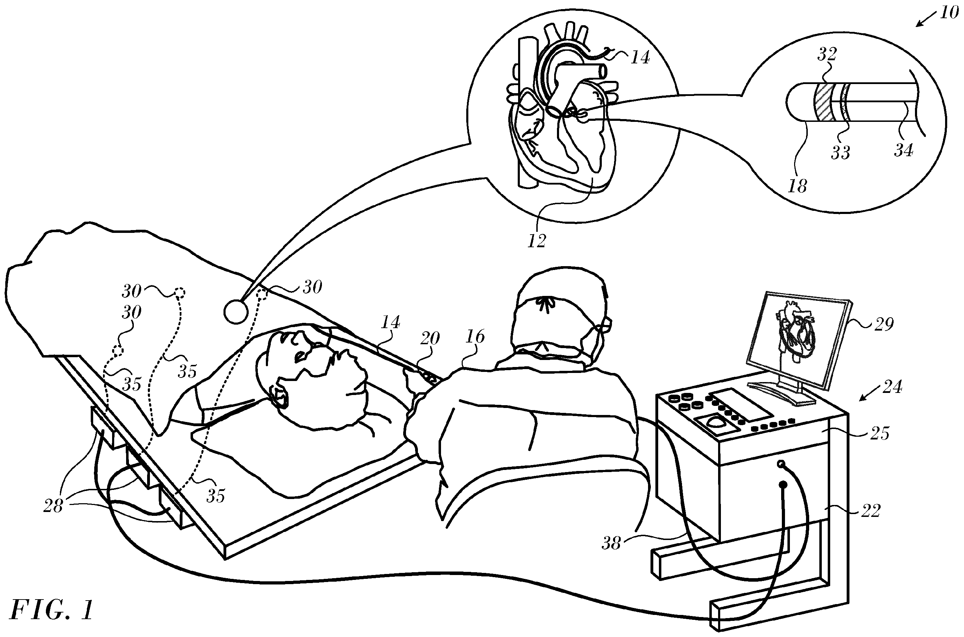

Turning now to the drawings, reference is initially made to Fig. 1, which is a pictorial illustration of a system 10 for evaluating electrical activity and performing ablative procedures on a heart 12 of a living subject, which is constructed and operative in accordance with a disclosed embodiment of the invention. The system comprises a catheter 14, which is percutaneously inserted by an operator 16 through the patient's vascular system into a chamber or vascular structure of the heart 12. The operator 16, who is typically a physician, brings the catheter's distal tip 18 into contact with the heart wall, for example, at an ablation target site. Electrical activation maps may be prepared, according to the methods disclosed in U.S. Patent Nos. 6,226,542, and 6,301,496, and in commonly assigned U.S. Patent No. 6,892,091. One commercial product embodying elements of the system 10 is available as the CARTO® 3 System, available from Biosense Webster, Inc., 3333 Diamond Canyon Road, Diamond Bar, CA 91765. This system may be modified by those skilled in the art to embody the principles of the invention described herein.

Areas determined to be abnormal, for example by evaluation of the electrical activation maps, can be ablated by application of thermal energy, e.g., by passage of radiofrequency electrical current through wires in the catheter to one or more electrodes at the distal tip 18, which apply the radiofrequency energy to the myocardium. The energy is absorbed in the tissue, heating it to a point (typically about 50°C) at which it permanently loses its electrical excitability. When successful, this procedure creates non-conducting lesions in the cardiac tissue, which disrupt the abnormal electrical pathway causing the arrhythmia. The principles of the invention can be applied to different heart chambers to diagnose and treat many different cardiac arrhythmias.

The catheter 14 typically comprises a handle 20, having suitable controls on the handle to enable the operator 16 to steer, position and orient the distal end of the catheter as desired for the ablation. To aid the operator 16, the distal portion of the catheter 14 contains position sensors (not shown) that provide signals to a processor 22, located in a console 24. The processor 22 may fulfill several processing functions as described below.

Ablation energy and electrical signals can be conveyed to and from the heart 12 through one or more ablation electrodes 32 located at or near the distal tip 18 via cable 34 to the console 24. Pacing signals and other control signals may be conveyed from the console 24 through the cable 34 and the electrodes 32 to the heart 12. Sensing electrodes 33, also connected to the console 24 are disposed between the ablation electrodes 32 and have connections to the cable 34.

Wire connections 35 link the console 24 with body surface electrodes 30 and other components of a positioning sub-system for measuring location and orientation coordinates of the catheter 14. The processor 22 or another processor (not shown) may be an element of the positioning subsystem. The electrodes 32 and the body surface electrodes 30 may be used to measure tissue impedance at the ablation site as taught in U.S. Patent No. 7,536,218, issued to Govari et al.*,* which is herein incorporated by reference. A temperature sensor (not shown), typically a thermocouple or thermistor, may be mounted on or near each of the electrodes 32.

The console 24 typically contains one or more ablation power generators 25. The catheter 14 may be adapted to conduct ablative energy to the heart using any known ablation technique, e.g., radiofrequency energy, ultrasound energy, and laser-produced light energy. Such methods are disclosed in commonly assigned U.S. Patent Nos. 6,814,733, 6,997,924, and 7,156,816.

In one embodiment, the positioning subsystem comprises a magnetic position tracking arrangement that determines the position and orientation of the catheter 14 by generating magnetic fields in a predefined working volume and sensing these fields at the catheter, using field generating coils 28. The positioning subsystem is described in U.S. Patent No. 7,756,576, and in the above-noted U.S. Patent No. 7,536,218.

As noted above, the catheter 14 is coupled to the console 24, which enables the operator 16 to observe and regulate the functions of the catheter 14. Console 24 includes a processor, preferably a computer with appropriate signal processing circuits. The processor is coupled to drive a monitor 29. The signal processing circuits typically receive, amplify, filter and digitize signals from the catheter 14, including signals generated by sensors such as electrical, temperature and contact force sensors, and a plurality of location sensing electrodes (not shown) located distally in the catheter 14. The digitized signals are received and used by the console 24 and the positioning system to compute the position and orientation of the catheter 14, and to analyze the electrical signals from the electrodes.

In order to generate electroanatomic maps, the processor 22 typically comprises an electroanatomic map generator, an image registration program, an image or data analysis program and a graphical user interface configured to present graphical information on the monitor 29.

Typically, the system 10 includes other elements, which are not shown in the figures for the sake of simplicity. For example, the system 10 may include an electrocardiogram (ECG) monitor, coupled to receive signals from one or more body surface electrodes, in order to provide an ECG synchronization signal to the console 24. As mentioned above, the system 10 typically also includes a reference position sensor, either on an externally-applied reference patch attached to the exterior of the subject's body, or on an internally-placed catheter, which is inserted into the heart 12 maintained in a fixed position relative to the heart 12. Conventional pumps and lines for circulating liquids through the catheter 14 for cooling the ablation site are provided. The system 10 may receive image data from an external imaging modality, such as an MRI unit or the like and includes image processors that can be incorporated in or invoked by the processor 22 for generating and displaying images.

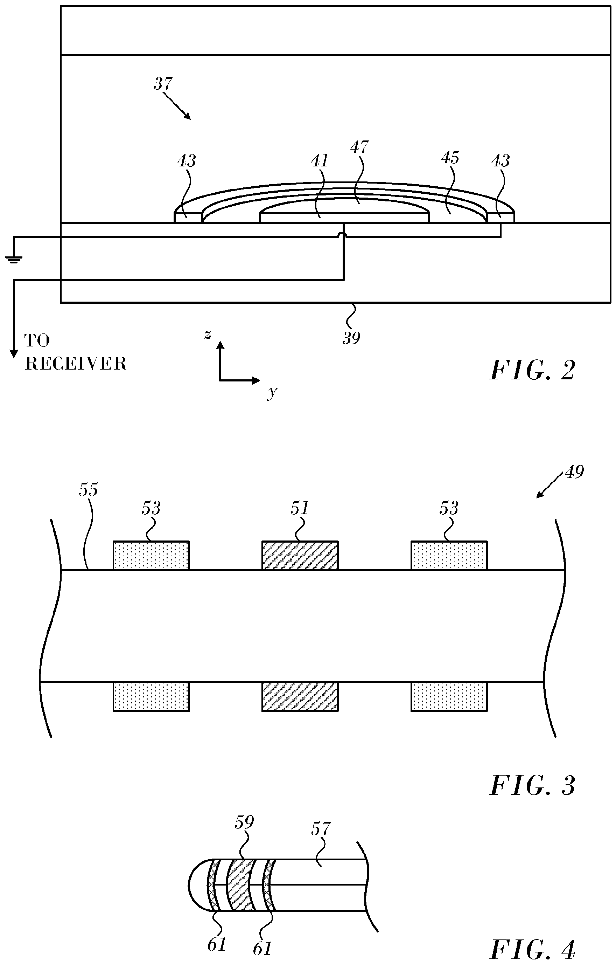

Reference is now made to Fig. 2, which is a schematic diagram of an electrode assembly 37 on the shaft of a cardiac catheter 39 in accordance with an embodiment of the invention. The electrode assembly 37 comprises a unipolar sensing electrode 41 bracketed on at least two sides by guard electrodes 43. A protective effect against far field interference is represented by an arcuate zone 45 that extends between the guard electrodes 43 and overarches the sensing electrode 41. Near-field potentials are detected by the sensing electrode 41 in a zone 47 immediately overlying the sensing electrode 41 and within the zone 45. The guard electrodes 43 are grounded. The sensing electrode 41 is connected to receiving circuitry that reads the potentials measured by the sensing electrode 41.

Reference is now made to Fig. 3, which is a schematic sectional view through the longitudinal axis of the distal portion of a cardiac catheter 49 in accordance with an embodiment of the invention. Fig. 3 illustrates a profile of sensing electrode 51 and guard electrodes 53. In practice the sensing electrode 51 and the guard electrodes 53 need not be raised above the surface of the catheter, but can be flush with the surface. In embodiments in which they are elevated above the surface, the elevation should be small, relative to the widths of the electrodes. The sensing electrode 51 and guard electrodes 53 are typically less than 0.3 mm in height. The heights and thicknesses of these electrodes are not critical.

Reference is now made to Fig. 4, which is a schematic side view of an electrode arrangement on the distal portion of a cardiac catheter 57 in accordance with an embodiment of the invention. A unipolar ring electrode 59 used for electrophysiological mapping of cardiac conduction lies between two grounded guard electrodes 61, which are also ring electrodes. As explained above in the discussion of Fig. 2, when the catheter is inserted into a heart and mapping is carried out, the guard electrodes 61 decrease far field signals at the ring electrode 59, i.e., signals originating at distances of 2 mm or more from the ring electrode 59. The guard electrodes 61 do not influence near field signals, i.e., those originating at distances less than 2 mm from the ring electrode 59. Optimum spacing between the ring electrode 59 and guard electrodes 61 varies with the desired distance range between the ring electrode 59 and target tissue. The interspaces are set such that signals originating from sources more remote than the desired distance range are considered as far field signals and reduced by the guard electrodes 61. In general it is suitable to adjust the gap and electrode dimensions such that the interspace is a half-width of the ring electrode 59

### Alternate Embodiment.

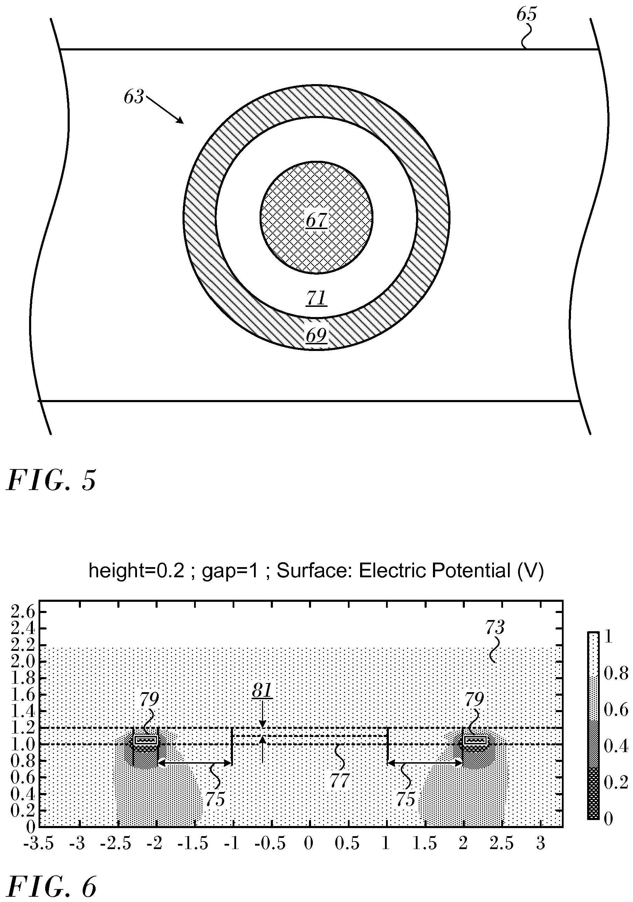

Reference is now made to Fig. 5, which is a schematic plan view of an electrode arrangement 63 on the distal portion of a cardiac catheter 65 in accordance with an alternate embodiment of the invention. A plate electrode 67 is connected to receiving circuitry. The electrode 67 is used for obtaining electrograms, e.g., for mapping purposes. The electrode 67 is encircled by guard electrode 69, which is connected to ground, as shown in Fig. 2. The electrode 67 and guard electrode 69 are spaced apart by a gap 71, which is typically 1 - 2 mm in width. The cardiac catheter 65 may include any number of instances of the electrode arrangement 63.

### Examples.

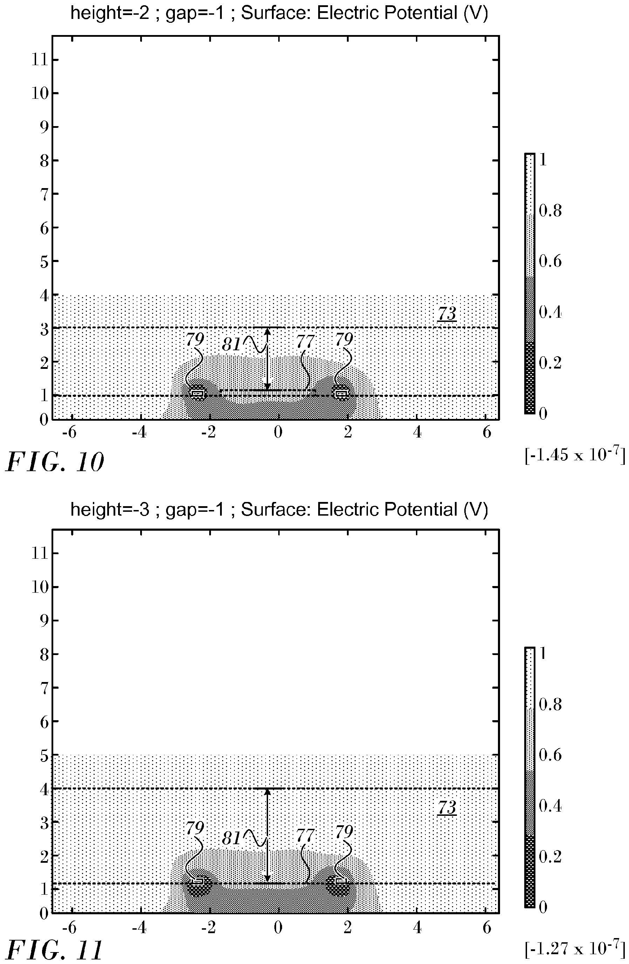

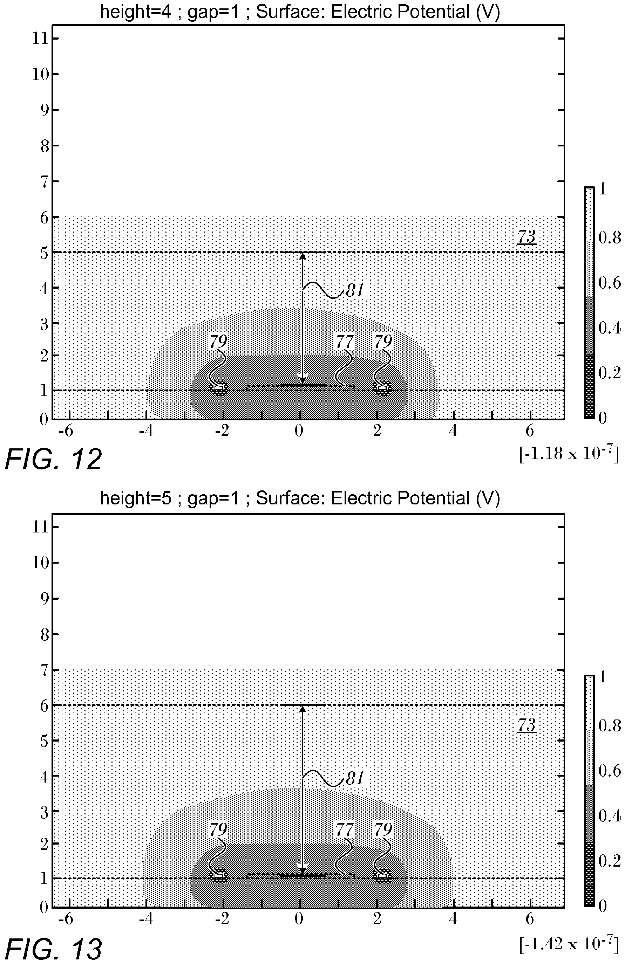

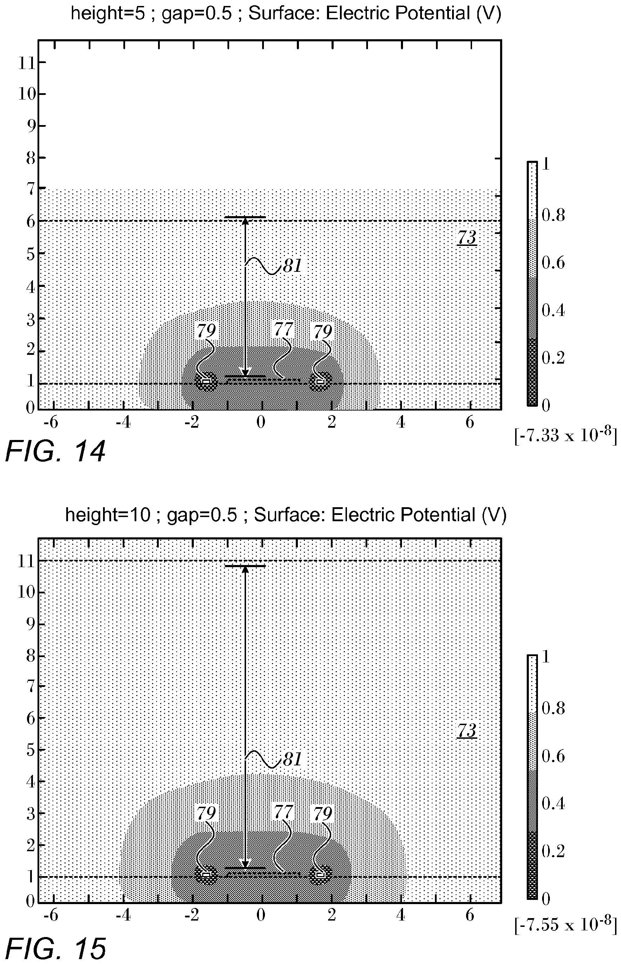

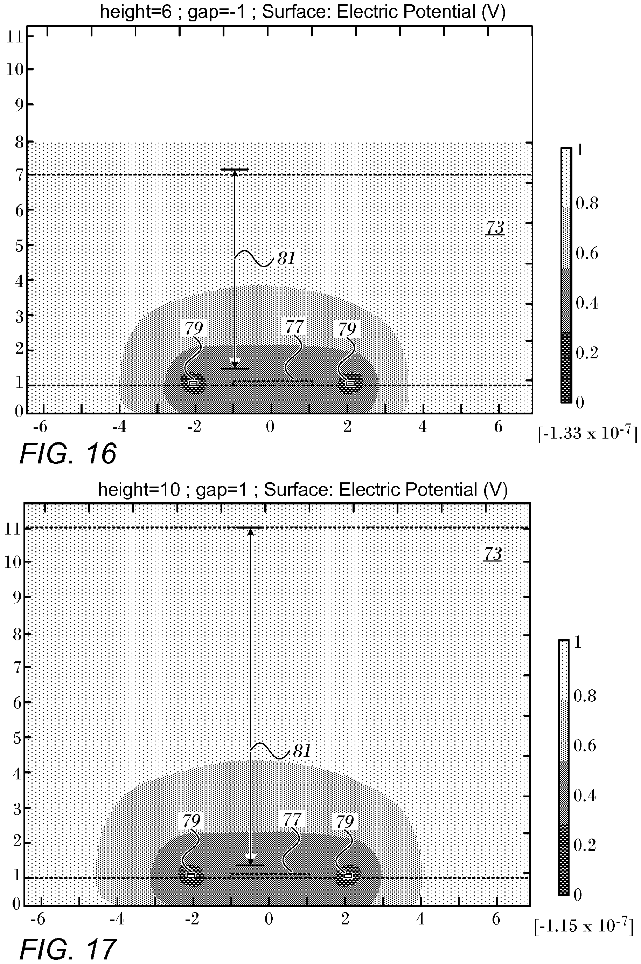

Fig. 6, Fig. 7, Fig. 8, Fig. 9, Fig. 10, Fig. 11, Fig. 12, Fig. 13, Fig. 14, Fig. 15, Fig. 16 and Fig. 17 are simulation examples showing the effect on the far field component of an intracardiac electrogram.as the height of the guard electrodes varies from 0.2 - 10 mm. In these simulations a plate 73 is the source of an electric field. Instances of a gap 75 that separates sensing electrode 77 and guard electrodes 79 are occur at 0.5 mm and 1 mm. Electrical field strength is indicated by the keys on a scale at the right of the drawing figures. The variation in the patterns illustrate the shielding effect of the guard electrodes 79. The field intensity of the plate 73 has a maximum intensity of 1. The guard electrodes 79, which are grounded, experience a minimum field intensity,

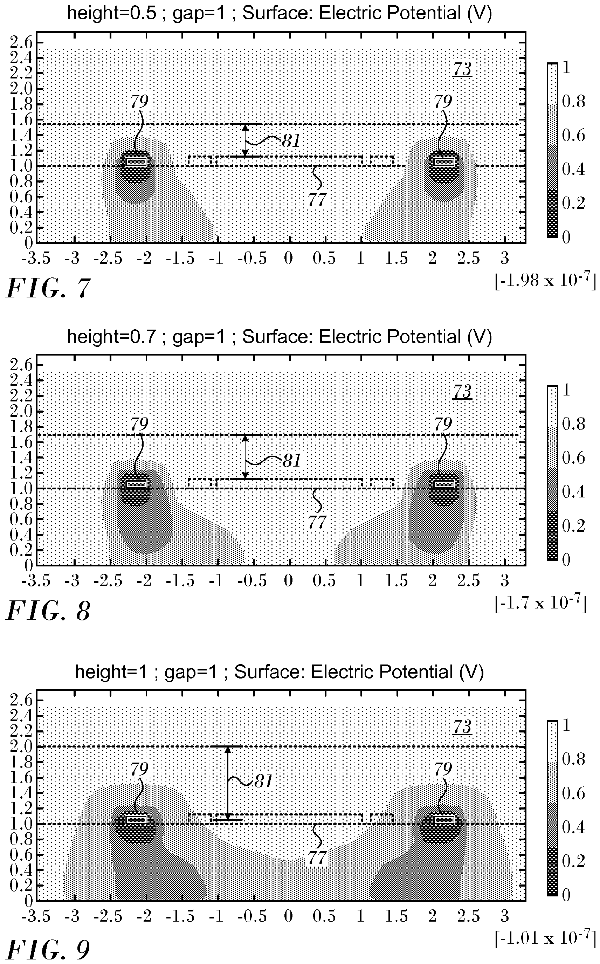

In the figures, especially Fig. 6, in which the plate 73 is close to the sensing electrode 77, the plate 73 acts as a near field source, and the sensing electrode 77 experiences a relatively high field intensity. The field intensity to which the sensing electrode 77 is exposed progressively diminishes in the examples of Fig. 7 - Fig. 12 as a distance 81 between the plate 73 and the sensing electrode 77 increases. The gap 75 between the sensing electrode 77 and guard electrodes 79 is optimally electrode half-width, i.e., half the width of the sensing electrode 77. A larger gap tends to decrease the screening effect, while a smaller gap decreases the signal of the sensing electrode 77.

The value scale at the right of the figures represents the relative strength of the electric field. The drawing figures illustrate the shielding effect of the guard electrode. The source of the field is the plate 73 in the upper portion of the figures. Its potential is the highest and is equal to 1 in the relative value scale. When the upper plate 73 is close to the sensing electrode 77, it acts as a near field source and the sensing electrode 77 experiences a high field value, i.e., it receives the full field strength of the plate 73. As the distance 81 between the plate 73 and the sensing electrode 77 increases, the field produced by the plate 73 increasingly simulates the characteristics of a far field source. The voltage received by the receiving electrode decreases and the electrode field strength at the sensing electrode 77 decreases accordingly. The dimensions of the sensing electrode 77 the guard electrodes 79 and the gap 75 all influence the field strength experienced by the sensing electrode 77.

The following configuration was used in the simulation examples: Plate receiving electrode (radius rplate=1mm), guard ring electrode (ring=1.4-2.5mm, ring width=0.3mm). Guard ring electrode grounded.

It will be appreciated by persons skilled in the art that the present invention is not limited to what has been particularly shown and described hereinabove. Rather, the scope of the present invention includes both combinations and sub-combinations of the various features described hereinabove, as well as variations and modifications thereof that are not in the prior art, which would occur to persons skilled in the art upon reading the foregoing description.

## Claims

1. An apparatus comprising:
a catheter (39, 49, 57, 65) having an elongated distal portion;
an electrode assembly (37, 63) disposed on the distal portion and comprising:
a sensing electrode (47, 51, 59, 67, 77) connected to a receiver; and
at least one grounded guard electrode (43, 53, 69, 79) spaced apart from the sensing electrode by a gap (45, 71, 75), the sensing electrode being bracketed on at least two sides by the at least one grounded guard electrode, wherein the sensing electrode has a width dimension, and **characterized in that** the gap is one-half the width dimension.

2. The apparatus according to claim 1, wherein the distal portion of the catheter has a circumference, and wherein the sensing electrode and the at least one grounded guard electrode are ring electrodes that encircle the circumference of the catheter.

3. The apparatus according to claim 1, wherein the catheter has an external surface, and the sensing electrode and the at least one grounded guard electrode are elevated above the external surface.

4. The apparatus according to claim 3, wherein the sensing electrode and the at least one grounded guard electrode have respective width dimensions, wherein elevations of the sensing electrode and the at least one grounded guard electrode are less than the respective width dimensions.

5. The apparatus according to claim 1, wherein the catheter has an external surface, and the sensing electrode and the at least one grounded guard electrode are flush with the external surface.

6. The apparatus according to claim 1, wherein the at least one grounded guard electrode is a single electrode that surrounds the sensing electrode.

## Patentansprüche

1. Vorrichtung, umfassend:
einen Katheter (39, 49, 57, 65) mit einem länglichen distalen Abschnitt;
eine Elektrodenanordnung (37, 63), die auf dem distalen Abschnitt angeordnet ist und umfasst:
eine Messelektrode (47, 51, 59, 67, 77), die mit einem Empfänger verbunden ist;
und
mindestens eine geerdete Schutzelektrode (43, 53, 69, 79), die von der Messelektrode durch einen Spalt (45, 71, 75) beabstandet ist, wobei die Messelektrode durch die mindestens eine geerdete Schutzelektrode auf mindestens zwei Seiten eingeklammert ist, wobei die Messelektrode eine Breitenabmessung aufweist, und **dadurch gekennzeichnet, dass**
der Spalt eine Hälfte der Breitenabmessung ist.

2. Vorrichtung nach Anspruch 1, wobei der distale Abschnitt des Katheters einen Umfang aufweist, und wobei die Messelektrode und die mindestens geerdete Schutzelektrode Ringelektroden sind, die den Umfang des Katheters umschließen.

3. Vorrichtung nach Anspruch 1, wobei der Katheter eine Außenfläche aufweist, und die Messelektrode und die mindestens eine geerdete Schutzelektrode über die Außenfläche erhöht sind.

4. Vorrichtung nach Anspruch 3, wobei die Messelektrode und die mindestens eine geerdete Schutzelektrode jeweilige Breitenabmessungen aufweisen, wobei Höhen der Messelektrode und der mindestens einen geerdeten Schutzelektrode geringer als die jeweiligen Breitenabmessungen sind.

5. Vorrichtung nach Anspruch 1, wobei der Katheter eine Außenfläche aufweist, und die Messelektrode und die mindestens eine geerdete Schutzelektrode bündig mit der Außenfläche abschließen.

6. Vorrichtung nach Anspruch 1, wobei die mindestens eine geerdete Schutzelektrode eine einzige Elektrode ist, welche die Messelektrode umgibt.

## Revendications

1. Appareil comprenant :
un cathéter (39, 49, 57, 65) ayant une partie distale allongée ;
un ensemble d'électrodes (37, 63) disposé sur la partie distale et comprenant :
une électrode de détection (47, 51, 59 ,67, 77) reliée à un récepteur ;
et
au moins une électrode de garde mise à la terre (43, 53, 69, 79) espacée de l'électrode de détection par un entrefer (45, 71, 75), l'électrode de détection étant entourée sur au moins deux côtés par l'au moins une électrode de garde mise à la terre, l'électrode de détection ayant une dimension dans la largeur, et **caractérisé en ce que**
l'entrefer représente la moitié de la dimension dans la largeur.

2. Appareil selon la revendication 1, dans lequel la partie distale du cathéter a une circonférence, et dans lequel l'électrode de détection et l'au moins une électrode de garde mise à la terre sont des électrodes annulaires qui encerclent la circonférence du cathéter.

3. Appareil selon la revendication 1, dans lequel le cathéter a une surface externe, et l'électrode de détection et l'au moins une électrode de garde mise à la terre sont surélevées par rapport à la surface externe.

4. Appareil selon la revendication 3, dans lequel l'électrode de détection et l'au moins une électrode de garde mise à la terre ont des dimensions dans la largeur respectives, les hauteurs de l'électrode de détection et de l'au moins une électrode de garde mise à la terre étant inférieures aux dimensions dans la largeur respectives.

5. Appareil selon la revendication 1, dans lequel le cathéter a une surface externe, et l'électrode de détection et l'au moins une électrode de garde mise à la terre sont au même niveau que la surface externe.

6. Appareil selon la revendication 1, dans lequel l'au moins une électrode de garde mise à la terre est une électrode unique qui entoure l'électrode de détection.
